# EUROPEAN PATENT APPLICATION

(11) **EP 4 008 769 A1**
(43) Date of publication of application: **08.06.2022**
(21) Application number: 21211431.8
(22) Date of filing: 30.11.2021
(51) Int. Cl.: C12M 1/107, C12M 1/02, C12M 1/00

(54) **DEVICE AND METHOD FOR THE PRODUCTION OF ELECTRICITY AND HEAT BY PROCESSING BIOMASS**

(30) Priority: 30.11.2020 BE 202005866
(71) Applicant: Krivalec bvba, 8610 Kortemark (BE)
(72) Inventor: Vansevenant, Kristof, 8610 Kortemark (BE)
(74) Representative: Brantsandpatents bvba

(57) **Abstract**

The present invention relates to an arrangement for producing electricity and heat comprising a fermentation tank, wherein the fermentation tank comprises an insulated bottom, insulated side walls, a gastight roof, a biomass inlet, a biogas outlet, a digestate outlet and heating pipes, and a cogeneration installation, comprising a gas engine and a generator, wherein the gas engine comprises a gas inlet and a motor shaft, the generator comprising a generator shaft, wherein the gas inlet is coupled to the biogas outlet and wherein the motor shaft is coupled to the generator shaft, wherein the gas engine comprises a cooling circuit, wherein the heating pipes of the fermentation tank are coupled to the cooling circuit of the engine. The invention also relates to a method for production of electricity and heat and to a use of an arrangement or method according to the present invention for production in an industrial company of electricity and heat from biomass.

## Description

### TECHNICAL FIELD

The invention relates to an arrangement for producing electricity and heat.

In a second aspect, the invention also relates to a method for producing electricity and heat.

In a third aspect, the invention also relates to a use for producing electricity and heat from biomass in an industrial company.

### PRIOR ART

In addition to products, various industries, such as farms and food companies, also produce waste streams such as animal manure and vegetable and fruit waste. For ecological reasons, these waste streams are no longer simply incinerated, dumped in a landfill or spread out on a field, for example. Today, these waste streams are regarded as valuable biomass, from which biogas can be obtained through fermentation. Such fermentation can take place locally, so that the biomass does not have to be transported. The biogas can then be used to produce electricity and heat. The electricity and heat produced is used, for example, on the farm, in a food company or other industrial company to carry out business activities.

The fermentation of biomass can be performed either as a low temperature mesophilic process, between 32°C and 42°C, or as a high temperature thermophilic process, between 48°C and 55°C. A mesophilic process is more robust than a thermophilic process but has the disadvantage that the fermentation of the biomass proceeds more slowly. An additional disadvantage is that a digestate, which remains as a residual product after fermentation, is in many cases not hygienic, as a result of which the digestate cannot simply be discharged or spread on a field and certainly cannot be exported. A specialized company has to collect the digestate for further processing, which leads to additional transport and processing costs for, for example, a farm, food company or other industrial company.

In a thermophilic process, the process is faster and pathogenic micro-organisms are killed faster. As a result, the digestate is hygienic and can, for example, be processed into fertilizer in a company without additional process steps for making the digestate hygienic. The digestate is a valuable raw material instead of a residual product. A particularly disadvantageous aspect of a thermophilic process is that the process has a very high energy requirement due to the high temperature. Another disadvantage is that the process is less robust. Minor temperature fluctuations can already disrupt the process. For these reasons, a mesophilic process is often preferred for fermentation.

The following methods and devices are known from the prior art:
WO 2004/035491 describes a method and a device for stabilization of a thermophilic temperature range and mineralization of sludge from wastewater purification installations.
US 2008/299634 discloses a biogas installation for producing biogas from biomass and methods of operating the biogas installation.
FR 2 991 993 is related to a system for combined production of e.g., electricity from agricultural waste, which has a control device for controlling the methanization unit and a cogeneration group and boiler for optimizing the production of energy within the system.

The present invention aims to solve at least some of the above problems or drawbacks.

### SUMMARY OF THE INVENTION

In a first aspect, the present invention relates to an arrangement according to claim 1.

The great advantage of such an arrangement is that a gas engine of a cogeneration installation comprises a cooling circuit, wherein heating pipes of a fermentation tank of the arrangement are coupled to the cooling circuit of the gas engine. This allows the fermentation tank to be heated in an energy efficient and economically favorable manner. In operation, the gas engine generates heat anyway, which would otherwise be absorbed and dissipated by the cooling circuit. Since the fermentation tank can be heated with the aid of this heat, a stable, high-temperature environment can be created in an arrangement according to the present invention, in which fermentation according to a thermophilic process can be performed robustly. Since a thermophilic process can be used in the arrangement according to the present invention, a hygienic digestate can be obtained, which can be sold as a valuable raw material.

Preferred forms of the arrangement are set out in claims 2 to 8.

In a specific preferred form, the invention relates to an arrangement according to claim 5.

In this preferred form, the arrangement comprises a separator configured to separate the digestate into a first thin and a first thick fraction. The first thick fraction can be used as fertilizer, making it a valuable product in its own right. It can optionally be spread locally as fertilizer on a field or sold as a valuable raw material to, for example, a fertilizer producer. Because the first thin fraction can be removed from the digestate, transport costs for transporting the first thick fraction to, for example, a fertilizer producer will be lower than the transport costs for transporting the digestate, as a result of which the first thick fraction is more valuable as a raw material than the digestate. Because the first thick fraction is obtained from a hygienic digestate, the first thick fraction is also hygienic, whereby the first thick fraction can be exported to countries where there is a shortage of fertilizers or whereby the thick first fraction can be used as cubicle bedding. Hygienic cubicle bedding reduces the risk of, for example, udder infections in cattle.

In a second aspect, the present invention relates to a method according to claim 9. This method has the advantage, among other things, that biomass is converted into biogas by means of a thermophilic process in a fermentation tank. The fermentation tank is heated to a high temperature with cooling water from the gas engine in an ecologically and economically efficient manner. Heating the fermentation tank has the advantage that a stable, high-temperature environment is created in which the thermophilic process can be performed robustly. An additional advantage is that a hygienic digestate is obtained, which can be sold as a valuable raw material.

Preferred embodiments of the method are described in the dependent claims 10 to 14.

In a third aspect, the present invention relates to a use according to claim 15. This use results in an advantageous production in an industrial company of electricity and heat from biomass. Biomass is converted into electricity and heat in an industrial company. Certain industrial companies, such as a farm or a food company, generate their own biomass in the form of manure, vegetable waste, fruit waste and/or other organic waste. The electricity and heat are used in all kinds of business activities in the industrial company. Remaining electricity and heat can be sold. Because it is hygienic, digestate can be sold to a fertilizer producing company as a valuable raw material or possibly processed into fertilizer in the industrial company. In the case of a farm, the fertilizer can be spread on its own fields for fertilization or sold as a valuable product. The biomass is converted on site into useful electricity, heat and valuable raw materials or products, which avoids additional transport and processing costs for the collection and processing of biomass and a non-hygienic digestate. Residual fractions, such as sludge, for example, are reused as biomass in the industrial company, so that the residual fractions do not have to be removed for processing.

### DESCRIPTION OF THE DRAWINGS

**Figure 1** shows a schematic representation of an arrangement according to an embodiment of the present invention.
**Figure 2** shows a schematic representation of an arrangement according to an alternative embodiment of the present invention.
**Figure 3** shows a schematic representation of an arrangement according to yet another alternative embodiment of the present invention.

### DETAILED DESCRIPTION

Unless otherwise defined, all terms used in the description of the invention, including technical and scientific terms, have the meaning as commonly understood by a person skilled in the art to which the invention pertains. For a better understanding of the description of the invention, the following terms are explained explicitly.

In this document, "a" and "the" refer to both the singular and the plural, unless the context presupposes otherwise. For example, "a segment" means one or more segments.

The terms "comprise", "comprising", "consist of", "consisting of", "provided with", "include", "including", "contain", "containing", are synonyms and are inclusive or open terms that indicate the presence of what follows, and which do not exclude or prevent the presence of other components, characteristics, elements, members, steps, as known from or disclosed in the prior art.

Quoting numerical intervals by endpoints comprises all integers, fractions and/or real numbers between the endpoints, these endpoints included.

In the context of this document, dry matter content of a solution is expressed as a mass of dry matter in kg in a volume of 1 m³ of the solution. The mass of dry matter is determined by taking a sample of known volume, drying the sample until all liquid has completely evaporated and only dry matter remains, weighing the mass of dry matter and recalculating the mass of dry matter in the known volume of the solution to a volume of 1 m³ of the solution. Alternatively, the dry matter content can also be expressed as a ratio of the mass of the dry matter in a known mass of the solution. This is represented as a dimensionless number or as a percentage.

In the context of this document, a cogeneration installation is an installation for the simultaneous generation of heat and power in the form of electricity, wherein the generated heat is usefully used locally, for example for the production of hot water, steam or hot air.

In the context of this document, a "spherical cap" is a part of a sphere that is cut off from the sphere by a plane.

In a first aspect, the invention relates to an arrangement for producing electricity and heat.

According to a preferred embodiment, the arrangement comprises a fermentation tank and a cogeneration installation.

The fermentation tank comprises an insulated bottom and insulated side walls. This is advantageous for preserving heat in the fermentation tank, so that there is a stable temperature in the fermentation tank, which is advantageous for a thermophilic fermentation process. The insulated bottom and the insulated side walls define an interior space. The interior space is suitable for the collection of biomass. Preferably, the insulated bottom and the insulated side walls are made of concrete. Preferably, the insulated bottom and insulated side walls form a cylindrical vessel with an open top. This is advantageous for obtaining a structurally strong fermentation tank that can absorb forces exerted by biomass in the fermentation tank.

The fermentation tank comprises a gastight roof configured to gas tightly seal the open top of the fermentation tank. The gastight roof is advantageous for storing biogas formed from biomass in the fermentation tank. The gastight roof preferably comprises a lung formed from a flexible and gastight sheet, and an overlying protective sheet, preferably the protective sheet only touching the lung near the insulated side walls. This is advantageous because the lung can expand when biogas is formed in the fermentation tank, while the protective sheet protects the lung against damage and consequently leakage. The gastight roof is preferably conical, wherein the base of the cone is at the open top of the fermentation tank and wherein the top of the cone is oriented in a direction away from the insulated bottom. Alternatively, the roof is a spherical cap, wherein the plane through which the spherical cap is cut from the sphere is at the open top of the fermentation tank and wherein the spherical cap is on one side of the plane away from the insulated bottom. The gastight roof is preferably centrally supported by a post, more preferably a hardwood post. A hardwood post is advantageous because such a post is resistant to harmful components that may be present in the biomass, digestate or biogas, such as, for example, but not limited to H₂S.

The fermentation tank comprises a biomass inlet. Biomass may comprise manure, vegetable waste, fruit waste and/or other organic waste. The inlet comprises a pump, an auger and/or other suitable means.

The fermentation tank comprises a biogas outlet. The biogas outlet comprises at least one gas pipe that is coupled to a gastight connection of the fermentation tank. The biogas outlet is preferably centrally located in the gastight roof. This is advantageous because biogas is lighter than air and rises to the gastight roof. A conical gastight roof is also advantageous for collecting the biogas at the top of the cone.

The fermentation tank comprises a digestate outlet. Digestate is a residual product that remains in the fermentation tank after the biomass has been decomposed. Digestate is liquid. The digestate outlet comprises an outlet tube. The digestate outlet preferably comprises a valve.

The fermentation tank comprises heating pipes. The heating pipes are preferably present over the entire perimeter of the insulated side walls. The heating pipes are preferably present at different heights on the insulated side walls. The heating pipes are advantageous for heating and maintaining the temperature of the fermentation tank and the biomass in the fermentation tank, allowing a stable environment at a suitable temperature for thermophilic fermentation. A suitable temperature is a temperature of at least 48°C and at most 55°C. Due to the insulated bottom and the insulated side walls, the heat required for heating the fermentation tank is limited compared to non-insulated fermentation tanks.

The cogeneration installation comprises a gas engine and a generator. The gas engine comprises a motor shaft. The gas engine is an internal combustion engine wherein said motor shaft is a crankshaft. Alternatively, the gas engine is a gas turbine, wherein the motor shaft is a shaft on which turbine blades are mounted. The gas engine comprises a gas inlet. The gas inlet is linked to the biogas outlet. This is advantageous in that the gas engine is thereby configured to operate on biogas produced by means of the arrangement. The generator comprises a generator shaft. The generator comprises a rotor and a stator. The generator shaft is contained within the rotor of the generator. The generator is configured to convert mechanical energy in the form of a rotating rotor into electrical energy. The motor shaft is coupled to the generator shaft. This is advantageous for converting a mechanical energy in the form of a rotating motor shaft to electrical energy. The motor shaft is coupled directly to the generator shaft. Alternatively, the motor shaft is coupled to the generator shaft via a transmission.

The gas engine comprises a cooling circuit. The cooling circuit is configured to cool the gas engine in operation. The heating pipes of the fermentation tank are connected to the cooling circuit of the gas engine. The heating pipes are connected directly to the cooling circuit. The heating pipes are preferably coupled to the cooling circuit via a heat exchanger. The coupling of the heating pipes to the cooling circuit of the gas engine is particularly advantageous in that as a result the fermentation tank can be heated in an energy-saving and economically favorable manner. In operation, the gas engine generates heat anyway, which would otherwise be absorbed and dissipated by the cooling circuit. Since the fermentation tank can be heated with the aid of this heat and kept at the right temperature, a stable environment at a high and suitable temperature can be created in an arrangement according to the present invention, in which fermentation according to a thermophilic process can be performed robustly. Because a thermophilic process can be used in the arrangement according to the present invention, a hygienic digestate can be obtained, which can be sold as valuable raw material, for instance to a producer of fertilizers.

According to an embodiment, the fermentation tank comprises an immersion mixer. This is advantageous for mixing supplied biomass and biomass present in the fermentation tank, in order to obtain optimum fermentation.

According to an embodiment, the fermentation tank comprises an overpressure and an underpressure protection. The overpressure and underpressure protection is a valve that automatically opens at a predetermined overpressure or underpressure in the fermentation tank relative to the environment and allows an excess of biogas to escape from the fermentation tank or allows air to enter the fermentation tank to remove an unsafe overpressure or underpressure inside the fermentation tank. This prevents damage to the gastight roof.

According to a preferred embodiment, the insulated bottom and the insulated side walls of the fermentation tank are insulated on one side facing the interior space. This is advantageous because insulating material with which the insulated bottom and the insulated side walls are insulated protects said bottom and side walls from harmful components that may be present in the biomass, digestate or biogas. As a result, it is not necessary to apply a protective coating to the insulated bottom and insulated side walls, as is the case with a prior art fermentation tank. It is also advantageous that, because the insulating material is arranged on the side of the insulated bottom and the insulated side walls, facing the interior space, the insulating material is protected against weather conditions and animals, as a result of which no additional protective materials, such as for instance panels, have to be fitted on a side of the insulated bottom and the insulated side walls, directed away from the interior space.

According to a further embodiment, the insulated bottom and the insulated side walls are insulated with PUR foam. This is advantageous because PUR foam can be applied quickly and without additional fasteners to the said bottom and side walls.

According to a preferred embodiment, the heating pipes are mounted on the insulated walls on a side facing the interior space of the fermentation tank. As a result, there is a direct and efficient transfer of heat to the biomass. This is particularly advantageous in a previously described embodiment wherein insulated walls are insulated on the side facing the interior space of the fermentation tank. Heating pipes incorporated in the insulated walls, for example, such as in fermentation tanks according to the prior art, could only transfer heat to the biomass to a limited extent due to the presence of insulation. The heating pipes are preferably stainless-steel pipes. Stainless steel tubes are advantageous for avoiding damage from harmful components that may be present in the biomass, digestate or biogas. Stainless steel pipes are also advantageous for rapid heat transfer due to a high thermal conductivity coefficient of stainless steel.

According to a preferred embodiment, the arrangement comprises a condenser configured for condensation of water vapor between the biogas outlet and the gas engine. This is advantageous for removing water vapor from the biogas, resulting in better operation of the gas engine.

According to a preferred embodiment, the arrangement comprises an activated carbon filter between the biogas outlet and the gas engine. An activated carbon filter is suitable for removing H₂S or other harmful components from the biogas, so that they cannot damage the gas engine.

According to a preferred embodiment, the arrangement comprises a heat exchanger. The heat exchanger is configured to exchange heat between two separate circuits, wherein a first circuit is connected to the biomass inlet of the fermentation tank and a second circuit is connected to the digestate outlet. Biomass introduced into the fermentation tank can therefore be preheated by high temperature digestate from the fermentation tank, so that less heat produced by the cogeneration installation is required for heating and maintaining the temperature of the fermentation tank. Saved heat can be sold or put to good use for other business activities. It is particularly advantageous that, due to the preheating, the temperature difference between biomass introduced into the fermentation tank and biomass in the fermentation tank is smaller, whereby the fermentation tank is a stable environment for bacteria necessary for the thermophilic process and the thermophilic process can be performed robustly. An additional advantage is that digestate can be cooled quickly with the aid of the heat exchanger, so that the digestate can be further processed immediately, without requiring a storage buffer in which the digestate can cool down.

According to a preferred embodiment, the gas engine comprises a valve control and the arrangement comprises a gas sensor. The gas sensor is configured to measure an amount of methane in the biogas. The valve control is communicatively connected to the gas sensor. The valve control is connected to the gas sensor wired and/or wirelessly. This allows the valve control to read measured values from the gas sensor about the amount of methane in the biogas. This embodiment is particularly advantageous with an internal combustion engine as a gas engine. An internal combustion engine comprises one or more cylinders, wherein a cylinder comprises at least an inlet and an outlet valve. The valve control is configured to control the opening and closing of inlet and outlet valves. The control is done according to a timing. Correct timing is essential for proper combustion of biogas in the internal combustion engine. The timing depends on the amount of methane gas in the biogas. By adjusting the timing by the valve control in proportion to the amount of methane gas in the biogas, an optimal operation of the gas engine can be obtained, wherein a gas engine can be operated from 40 vol.% methane gas in biogas. The valve control comprises at least three discrete timing settings depending on the amount of methane gas in the biogas. The timing is preferably continuously adjustable by the valve control in proportion to the amount of methane gas in the biogas.

According to a preferred embodiment, the arrangement comprises a separator configured to separate the digestate into a first thin fraction and a first thick fraction.

The first thin fraction has a dry matter content of at most 50 kg/m³, preferably at most 48 kg/m³, more preferably at most 45 kg/m³, even more preferably at most 43 kg/m³ and even more preferably at most 40 kg/m³.

The first thick fraction has a dry matter content of at least 200 kg/m³, preferably at least 210 kg/m³, more preferably at least 220 kg/m³, even more preferably at least 230 kg/m³ and even more preferably at least 240 kg/m³.

The separator is, for example, a centrifuge, a belt press filter, a screw press or other suitable means. It will be apparent to one skilled in the art that a combination of several of said examples also forms part of the present invention.

Optionally, the separator comprises means configured for adding coagulant and/or flocculant to the digestate. This is advantageous for obtaining a better separation into a first thin fraction and a second thin fraction.

This embodiment is advantageous in that it allows the dry matter content in the first thick fraction to be increased relative to the dry matter content in the digestate, allowing the first thick fraction to be used as fertilizer and making it a valuable product in its own right. It can optionally be spread locally as fertilizer on a field or sold as a valuable raw material to, for example, a fertilizer producer. Because the first thin fraction can be removed from the digestate, transport costs for transporting the first thick fraction to, for example, a fertilizer producer will be lower than the transport costs for transporting the digestate, as a result of which the first thick fraction is more valuable as a raw material than the digestate. Because the first thick fraction is obtained from a hygienic digestate, the first thick fraction is also hygienic, whereby the first thick fraction can be exported to countries where there is a shortage of fertilizers or whereby the thick first fraction can be used as cubicle bedding. Hygienic cubicle bedding reduces the risk of, for example, udder infections in cattle.

According to a further embodiment, the arrangement comprises a purification installation configured to purify the first thin fraction. The purification installation comprises a dissolved air flotation installation, an optional ultrafiltration installation and a reverse osmosis installation.

A dissolved air flotation installation comprises a bath for the collection of a thin fraction. The bath preferably extends in a longitudinal direction. The bath comprises a supply, wherein the supply is configured for feeding thin fraction into the bath and the supply being preferably positioned at a first end along the longitudinal direction of the bath. The dissolved air flotation installation comprises means for supplying a coagulant to the thin fraction. The dissolved air flotation installation optionally comprises a supply for a base or an acid. This is advantageous for pH correction of coagulant treated thin fraction. Preferably, the dissolved air flotation installation comprises a mixer configured for mixing the thin fraction and the coagulant. The bath is at atmospheric pressure. Non-limiting examples of coagulant are FeCl₃ and Fe₂(SO₄)₃. Coagulant is advantageous for flocculation by destabilizing colloidal particles in the thin fraction. The dissolved air flotation installation optionally comprises a flocculant supply. Flocculant is advantageous for further enlarging flocs formed by coagulant. Flocculant is added together with coagulant or after addition of coagulant. The dissolved air flotation installation comprises a pressure vessel. The pressure vessel is configured to contain water at a pressure of at least 3 bar, preferably at least 4 bar, and more preferably at least 5 bar. The pressure vessel comprises means for introducing air into the pressure vessel. The dissolved air flotation installation comprises a pipe from the pressure vessel to the bath. By introducing water with air under pressure into the bath, air bubbles are formed in the bath at atmospheric pressure. These air bubbles cause light floc particles to float in the bath. Heavy floc particles settle in the bath. The bath comprises an outlet configured to discharge settled floc particles from the bath. The dissolved air flotation installation comprises means configured to remove the floating floc particles. A non-limiting example is a scraper configured for scraping floating floc particles from the bath. The bath comprises an outlet configured to drain liquid, wherein the liquid is substantially purified of floc particles. The outlet is preferably positioned at a second end along the longitudinal direction of the bath, wherein the second end is opposite the first end. The settled floc particles and the floating floc particles are sludge from the dissolved air flotation installation. The sludge from the dissolved air flotation installation is also advantageous as biomass in the fermentation tank.

The purification installation comprises pipes for carrying the liquid from the dissolved air flotation installation to the optional ultrafiltration installation. The pipes can carry the liquid directly to the optional ultrafiltration installation or indirectly through additional liquid purification installations and/or a buffer tank. The ultrafiltration installation comprises filter membranes. The filter membranes are installed dry. The ultrafiltration installation comprises a pump configured to pump the liquid under pressure from a first side to a second side through the filter membranes. The ultrafiltration installation comprises a liquid outlet on the second side. An ultrafiltration installation is advantageous for removing residual undissolved matter and/or macromolecules in a liquid, so that semipermeable membranes in a reverse osmosis installation have to be replaced or cleaned less often. The remaining undissolved matter and/or macromolecules that have been filtered from the liquid from the dissolved air flotation installation by the ultrafiltration installation is upgraded sludge from the ultrafiltration installation. The sludge from the ultrafiltration installation is also advantageous as biomass in the fermentation tank.

The purification installation comprises pipes for carrying the liquid from the outlet on the second side of the optional ultrafiltration installation to the reverse osmosis installation. If the water purification installation does not comprise an ultrafiltration installation, the purification installation comprises pipes for carrying the liquid from the dissolved air flotation installation to the reverse osmosis installation. The pipes can carry the liquid directly to the reverse osmosis installation or indirectly via additional liquid purification installations, such as for example a paper belt filter and/or a buffer tank. The reverse osmosis installation comprises a semipermeable membrane. The reverse osmosis installation comprises a liquid inlet and a first liquid outlet on a first side of the semipermeable membrane. The reverse osmosis installation comprises a second liquid outlet on a second opposite side of the semipermeable membrane. The reverse osmosis installation comprises a pump configured to pump under pressure the liquid from the outlet on the second side of the optional ultrafiltration installation or the liquid from the dissolved air flotation installation outlet into the liquid inlet. The pressure is at least higher than an osmotic pressure in the liquid. This causes water to migrate through the semipermeable membrane from the first side to the second side. Liquid with an increased concentration of minerals is discharged via the first outlet and purified water is discharged via the second outlet.

This embodiment is particularly advantageous for purifying thin fraction into purified water and in a mineral concentrate. The purified water is suitable as irrigation water. The purified water can also be discharged. The purified water can also be made suitable for use, for example, as process water in a food company, for example for rinsing and/or blanching vegetables. Due to the increased concentration of minerals in the mineral concentrate, the mineral concentrate is more valuable than a thin fraction, for example for a fertilizer producer. Because purified water has been removed from the mineral concentrate, transport costs for the mineral concentrate are lower than for the thin fraction.

According to an embodiment, the purification installation comprises an aeration tank and optionally a settling tank. The aeration tank comprises an inlet configured for feeding thin fraction into the aeration tank. The thin fraction comprises at least the first thin fraction. The aeration tank comprises an outlet configured to discharge thin fraction from the aeration tank. The outlet is coupled to the inlet of the dissolved air flotation installation, as in a previously described embodiment. The aeration tank comprises an outlet for settled sludge. The settled sludge is formed by suspended particles in the thin fraction that settle in the aeration tank. The settled sludge also comprises biological sludge generated by microbiological purification of thin fraction in the aeration tank. The disposal of sludge is advantageous to avoid completely filling the aeration tank with settled sludge and thus destroying biological activity in the aeration tank. The sludge from the aeration tank is also advantageous as biomass in the fermentation tank. The aeration tank comprises an aeration system, such as, for example, but not limited to aeration discs, tube aerators and/or immersion aerators. The aeration system is configured to create fine air bubbles in the thin fraction. An aeration tank is advantageous for removing nitrogen from the first thin fraction. This is done by converting ammonium to nitrate during nitrification and by converting nitrate to nitrogen gas during denitrification. The aeration tank comprises two compartments. In a first compartment, there is aeration. The first compartment is suitable for nitrification. In a second compartment, there is no aeration. The second compartment is suitable for denitrification. Alternatively, the aeration tank comprises a single compartment and the aeration system comprises a timed control, suitable for timed switching on or off of the aeration system. This is advantageous for nitrification during switched on aeration and denitrification during switched off aeration in a single compartment.

Optionally, the outlet is not directly linked to the inlet of the dissolved air flotation installation, but optionally a settling tank is incorporated in the purification installation between the aeration tank and the dissolved air flotation installation. A settling tank is advantageous for allowing sludge formed in the aeration tank to settle. This simplifies further purification of the thin fraction. The sludge from the settling tank is also advantageous as biomass in the fermentation tank.

According to a further embodiment, the aeration tank comprises heat pipes. The heat pipes are coupled to the cogeneration installation. The heat pipes are configured for heating by heat produced by the cogeneration installation, for example hot flue gases from the gas engine. The heat pipes are advantageous for a constant temperature in the aeration tank during the year, so that aerobic processes in the aeration tank are not disturbed by changing temperatures throughout the seasons. The heat pipes are advantageous for ecological and economical heating of the aeration tank.

According to an embodiment, the aeration system of the aeration tank comprises a heat exchanger, wherein the heat exchanger is configured to exchange heat between two separate circuits, wherein a first circuit is connected to the aeration system of the aeration tank. Air is compressed by the aeration system during aeration of the aeration tank, causing the air to warm up considerably. The air can heat up to a temperature of 80°C. Heat can be extracted from the air by the heat exchanger, which cools the air. The heat extracted can be usefully used in the second circuit to, for example, heat air or water.

According to a further embodiment, the second circuit of the heat exchanger is configured to preheat biomass before feeding into the fermentation tank. Biomass introduced into the fermentation tank is thereby preheated, so that less heat produced by the cogeneration installation is required for heating the fermentation tank. Saved heat can be sold or put to good use for other business activities. It is particularly advantageous that, due to the preheating, the temperature difference between biomass introduced into the fermentation tank and biomass in the fermentation tank is smaller, whereby the fermentation tank is a stable environment for bacteria necessary for the thermophilic process and the thermophilic process can be performed robustly.

It will be apparent to one skilled in the art that this embodiment can be combined with a heat exchanger configured for preheating the biomass by the digestate, as in a previously described embodiment.

According to an alternative embodiment, the second circuit of the heat exchanger is configured for heating the fermentation tank. The second circuit is coupled to heating pipes in the fermentation tank. This is advantageous because as a result less heat produced by the cogeneration installation is required for heating the fermentation tank. Saved heat can be sold or put to good use for other business activities. This is particularly advantageous if the fermentation tank cannot be heated sufficiently by the cooling circuit of the gas engine.

According to a preferred embodiment, the purification installation comprises a separator configured to separate sludge from the purification installation into a second thin and a second thick fraction. The separator is analogous to the separator for separating the digestate into a first thin fraction and a first thick fraction as in a previously described embodiment. The second thin fraction has a similar dry matter content as the first thin fraction. The second thick fraction has a similar dry matter content as the first thick fraction. The sludge from the purification installation is sludge from an aeration tank and/or from a settling tank and/or from a dissolved air flotation installation and/or from an ultrafiltration installation. The sludge from the purification installation has a dry matter content. The separator is advantageous for separating the sludge into a second thick fraction with a dry matter content higher than the sludge from the purification installation, as a result of which the second thick fraction is more advantageous as biomass in the fermentation tank than the sludge from the purification installation. The separator comprises a return pipe for the second thin fraction to the purification installation. The return pipe returns the second thin fraction to the purification installation for further purification of the second thin fraction into purified water and mineral concentrate as in a previously described embodiment. It will be apparent to one skilled in the art that in previously or hereinafter described embodiments of the purification installation, wherein the purification installation comprises a separator according to this embodiment, by "thin fraction" is meant both the first thin fraction and the second thin fraction. This embodiment is advantageous because the second thin fraction does not have to be removed for further processing, but is locally purified, which saves processing and transport costs.

According to an alternative embodiment, the arrangement comprises a purification installation configured to purify the first thin fraction, wherein the purification installation comprises an aeration tank, a membrane bioreactor, a reverse osmosis installation and optionally an ultrafiltration installation. The aeration tank, the optional ultrafiltration installation and the reverse osmosis installation are as in previously described embodiments and are interconnected as in these embodiments. The membrane bioreactor is an alternative to the settling tank and the air flotation installation in these previously described embodiments.

The membrane bioreactor comprises a bath configured to collect liquid from the aeration tank. Membranes are positioned in the bath. The membranes form a volume. The membranes comprise pores. The membrane bioreactor comprises a pump configured to draw liquid from the bath through the pores into the volume formed by the membranes. The pump has a flow rate. The flow rate is lower than a flow rate at which liquid flows from the aeration tank into the bath. The membrane bioreactor has an outlet configured for removal of drawn off liquid. The purification installation comprises pipes for carrying the liquid from the membrane bioreactor to the optional ultrafiltration installation. The pipes can carry the liquid directly to the ultrafiltration installation or indirectly through additional liquid purification installations and/or a buffer tank. If the purification installation does not comprise an ultrafiltration installation, the purification installation comprises pipes for carrying the liquid from the membrane bioreactor to the reverse osmosis installation. The pipes can carry the liquid directly to the reverse osmosis installation or indirectly through additional liquid purification installations and/or a buffer tank. The membrane bioreactor comprises an overflow. The overflow is advantageous for discharge of excess liquid in the membrane bioreactor. The excess liquid that flows out of the membrane bioreactor via the overflow has a higher dry matter content due to the suction of liquid through the membranes. The excess liquid is sludge from the membrane bioreactor. The sludge from the membrane bioreactor is advantageous as biomass in the fermentation tank. It will be apparent to one skilled in the art that the sludge can be separated into a second thin fraction and a second thick fraction by means of a separator as in a previously described embodiment with similar advantages and that the second thin fraction can be further purified in the purification installation by means of a return pipe. Alternatively, the purification installation comprises a return pipe configured to return the sludge from the membrane bioreactor to the aeration tank. It will be apparent to one skilled in the art that part of the sludge can be fed to a separator for separation into a second thin fraction and a second thick fraction and that part of the sludge can be fed back to the aeration tank via the return pipe.

According to an embodiment, the purification installation comprises an ion exchanger. The ion exchanger is connected to the second outlet of the reverse osmosis installation by means of pipes. The ion exchanger comprises synthetic resin. The ion exchanger is advantageous for removing unwanted ions from the purified water.

According to a preferred embodiment, the arrangement comprises transport means configured for automatically transporting sludge and/or second thick fraction from the purification installation to the fermentation tank. This is advantageous because the sludge or the second thick fraction has sufficient nutritional value to be suitable as biomass in the fermentation tank. This avoids having to remove the sludge and/or the second thick fraction for further processing, saving transport and processing costs. This is also advantageous because the sludge and/or the second fraction may still comprise a portion of unused coagulant, as in a previously described embodiment. Fe salts, as a non-limiting example, are particularly advantageous for converting H₂S to non-harmful sulfur forms, such as S or Fe₂S₃, which prevents damage to, for example, the fermentation tank and the cogeneration installation by H₂S.

According to a preferred embodiment, the arrangement comprises heating means configured for drying the first thick fraction, wherein the heating means are configured for use of heat produced by the cogeneration installation, for example hot flue gases from the gas engine. This is advantageous because it allows the first thick fraction to be dried in an ecological and economical manner. A non-limiting example is a drying auger. This embodiment is advantageous for further increasing the dry matter content of the first thick fraction. This makes the dried thick fraction even more valuable as fertilizer.

According to a preferred embodiment, the arrangement comprises a burner, wherein the burner is coupled to the biogas outlet. This embodiment is advantageous if the fermentation tank produces more biogas than required for the cogeneration installation. The burner converts biogas into heat that can be usefully used in various business activities or that can be sold. This embodiment is particularly advantageous as a heating means configured for drying the first thick fraction, as in a previously described embodiment.

According to a preferred embodiment, the arrangement comprises a pellet press configured for pressing dried first thick fraction into pellets. This is advantageous because the pellets can be easily stored. The pellets can be sold as fertilizer.

According to a preferred embodiment, the arrangement comprises a separator configured for separating the biomass into a third thin fraction and a third thick fraction. The third thin fraction and the third thick fraction are similar to the first thin fraction and the first thick fraction. The third thick fraction has a higher dry matter content than the biomass. The third thick fraction is therefore more advantageous than the biomass for the production of biogas, because the third thick fraction comprises less water, from which no biogas can be produced. The third thin fraction can be purified together with the first thin fraction in the purification installation for the first thin fraction. It will be apparent to one skilled in the art that in this embodiment, a thin fraction mentioned in the previously described embodiments of the purification installation, means the first thin fraction and/or second thin fraction and/or third thin fraction.

According to a further embodiment, the separator for separating the biomass into a third thin fraction and a third thick fraction is the separator for separating sludge from the purification installation into a second thin fraction and a second thick fraction. This is advantageous because in an arrangement which comprises the separator for separating sludge from the purification installation, a separator for separating biomass can be omitted. It will be apparent to one skilled in the art that in this embodiment the third thin fraction is equal to the second thin fraction and the third thick fraction is equal to the second thick fraction. This embodiment is preferably combined with a previously described embodiment wherein the arrangement comprises transport means configured for automatically transporting sludge and/or second thick fraction from the purification installation to the fermentation tank. This is advantageous because the third thick fraction is still made hygienically in the fermentation tank, whereby a hygienic first thick fraction can still be obtained according to previously described embodiments.

In a second aspect, the invention relates to a method for producing electricity and heat.

In a preferred embodiment, the method comprises the steps of:
- feeding biomass into a fermentation tank,
- fermentation of the biomass in the fermentation tank for at least fifty days at a temperature of at least 48°C and at most 55°C,
- discharging digestate from the fermentation tank,
- discharging biogas from the fermentation tank,
- generating heat and electricity from the biogas in a cogeneration installation, comprising a gas engine and a generator.

Fermenting biomass in the fermentation tank for at least fifty days at a temperature of at least 48°C and at most 55°C, preferably at least 49°C and at most 54°C, more preferably at least 50°C and at most 53°C, guarantees that the biomass ferments at a high temperature for a sufficient time by means of a thermophilic process, so that a hygienic digestate and biogas are obtained. Fifty days is a minimum duration according to applicable French law.

The fermentation tank is heated with cooling water from the gas engine. As a result, the fermentation tank is heated to a high temperature with cooling water from the gas engine in an ecologically and economically efficient manner. Heating the fermentation tank has the advantage that a stable, high-temperature environment is created in which the thermophilic process can be performed robustly. An additional advantage is that the hygienic digestate can be sold as a valuable raw material for fertilizer production.

According to a preferred embodiment, the method comprises the additional step of preheating the biomass by the digestate by means of a heat exchanger before feeding the biomass into the fermentation tank. Biomass introduced into the fermentation tank is preheated by high temperature digestate from the fermentation tank, so that less heat produced by the cogeneration installation is required for heating the fermentation tank. Saved heat can be sold or put to good use for other business activities. It is particularly advantageous that, due to the preheating, the temperature difference between biomass introduced into the fermentation tank and biomass in the fermentation tank is smaller, whereby the fermentation tank is a stable environment for bacteria necessary for the thermophilic process and the thermophilic process can be performed robustly. It is also advantageous that digestate is cooled quickly with the aid of the heat exchanger, preferably to a temperature below 40°C, so that the digestate can be further processed immediately, without requiring a storage buffer in which the digestate has to cool down. This is particularly advantageous for avoiding damage to e.g., membranes and/or filters of a purification installation, such as in previously described embodiments of an arrangement according to the present invention. Such membranes and filters are often only resistant to temperatures of up to 40°C. This is also particularly advantageous when using an aeration tank, such as in previously described embodiments of an arrangement according to the present invention, where temperatures above 40°C can disrupt aerobic processes in the aeration tank.

According to a preferred embodiment, the method comprises the additional step of separating the digestate using a separator into a first thin fraction and a first thick fraction.

The first thin fraction has a dry matter content of at most 50 kg/m³, preferably at most 48 kg/m³, more preferably at most 45 kg/m³, even more preferably at most 43 kg/m³ and even more preferably at most 40 kg/m³.

The first thick fraction has a dry matter content of at least 200 kg/m³, preferably at least 210 kg/m³, more preferably at least 220 kg/m³, even more preferably at least 230 kg/m³ and even more preferably at least 240 kg/m³.

This embodiment is advantageous in that the dry matter content in the first thick fraction relative to the dry matter content in the digestate is increased, allowing the first thick fraction to be used as fertilizer and making it a valuable product in its own right. It can optionally be spread locally as fertilizer on a field or sold as a valuable raw material to, for example, a fertilizer producer. Because the first thin fraction has been removed from the digestate, transport costs for transporting the first thick fraction to, for example, a fertilizer producer are lower than the transport costs for transporting the digestate, as a result of which the first thick fraction is more valuable as a raw material than the digestate. Because the first thick fraction is obtained from a hygienic digestate, the first thick fraction is also hygienic, whereby the first thick fraction can be exported to countries where there is a shortage of fertilizers or whereby the thick first fraction can be used as cubicle bedding. Hygienic cubicle bedding reduces the risk of, for example, udder infections in cattle.

According to a further embodiment, the method comprises the additional step of purifying the first thin fraction in a purification installation, wherein water, mineral concentrate and a residual fraction are obtained. The residual fraction is similar to sludge or a thick second fraction from a purification installation according to previously described embodiments of an arrangement according to the present invention.

The obtained water is suitable as irrigation water. The obtained water can also be discharged. The purified water can also be made suitable for use, for example, as process water in a food company, for example for rinsing and/or blanching vegetables. Due to an increased concentration of minerals in the obtained mineral concentrate, the obtained mineral concentrate is more valuable than a thin fraction, for example for a fertilizer producer. A thin fraction is similar to a thin fraction as described in the embodiments of an arrangement according to the present invention. Because water obtained from the mineral concentrate has been removed, transport costs for the mineral concentrate are lower than for thin fractions. The residual fraction is advantageous as biomass in the fermentation tank.

According to a further embodiment, the method comprises the additional step of returning the residual fraction to the fermentation tank. This is advantageous because the residual fraction has sufficient nutritional value to be suitable as biomass in the fermentation tank. This avoids having to remove the residual fraction for further processing, saving transport and processing costs. This is also advantageous because the residual fraction can still comprise a part of unused coagulant. Fe salts as a non-limiting example are particularly advantageous for conversion of H₂S to non-harmful sulfur compounds, such as for example S or Fe₂S₃, which prevents damage to, for example, the fermentation tank and the cogeneration installation by H₂S.

According to a preferred embodiment, the method comprises the additional step of separating the biomass using a separator into a third thin fraction and a third thick fraction if the biomass has a dry matter content of less than 150 kg/m³, preferably less than 140 kg/m³, more preferably less than 130 kg/m³, even more preferably less than 120 kg/m³ and even more preferably less than 110 kg/m³.

The third thin fraction has a dry matter content of at most 50 kg/m³, preferably at most 48 kg/m³, more preferably at most 45 kg/m³, even more preferably at most 43 kg/m³ and even more preferably at most 40 kg/m³.

The third thick fraction has a dry matter content of at least 200 kg/m³, preferably at least 210 kg/m³, more preferably at least 220 kg/m³, even more preferably at least 230 kg/m³ and even more preferably at least 240 kg/m³.

The third thick fraction has a higher dry matter content than the biomass. The third thick fraction is therefore more advantageous than the biomass for the production of biogas, because the third thick fraction comprises less water, from which no biogas can be produced. The third thick fraction is fed into the fermentation tank instead of the biomass. The third thin fraction is purified in the purification installation together with the first thin fraction.

One skilled in the art will appreciate that a method according to the second aspect is preferably performed using an arrangement according to the first aspect and that an arrangement according to the first aspect is preferably configured for performing a method according to the second aspect. Each feature described in this document, both above and below, can therefore relate to any of the three aspects of the present invention.

In a third aspect, the invention relates to a use of an arrangement according to the first aspect or a method according to the second aspect for production in an industrial company of electricity and heat from biomass.

This use results in an advantageous production in an industrial company of electricity and heat from biomass. A farm generates its own biomass in the form of manure, vegetable waste and/or fruit waste. Biomass is converted into electricity and heat in an industrial company. Certain industrial companies, such as a farm or a food company, generate their own biomass in the form of manure, vegetable waste, fruit waste and/or other organic waste. The electricity and heat are used in all kinds of business activities in the industrial company. Remaining electricity and heat can be sold. Because it is hygienic, digestate can be sold to a fertilizer producing company as a valuable raw material or possibly processed into fertilizer in the industrial company. In the case of a farm, the fertilizer can be spread on its own fields for fertilization or sold as a valuable product. The biomass is converted on site into useful electricity, heat and valuable raw materials or valuable products, which avoids additional transport and processing costs for the collection and processing of biomass and a non-hygienic digestate. Residual fractions, such as sludge, for example, are reused as biomass in the industrial company, so that the residual fractions do not have to be removed for processing.

In what follows, the invention is described by way of non-limiting figures illustrating the invention, and which are not intended to and should not be interpreted as limiting the scope of the invention.

### DESCRIPTION OF THE DRAWINGS

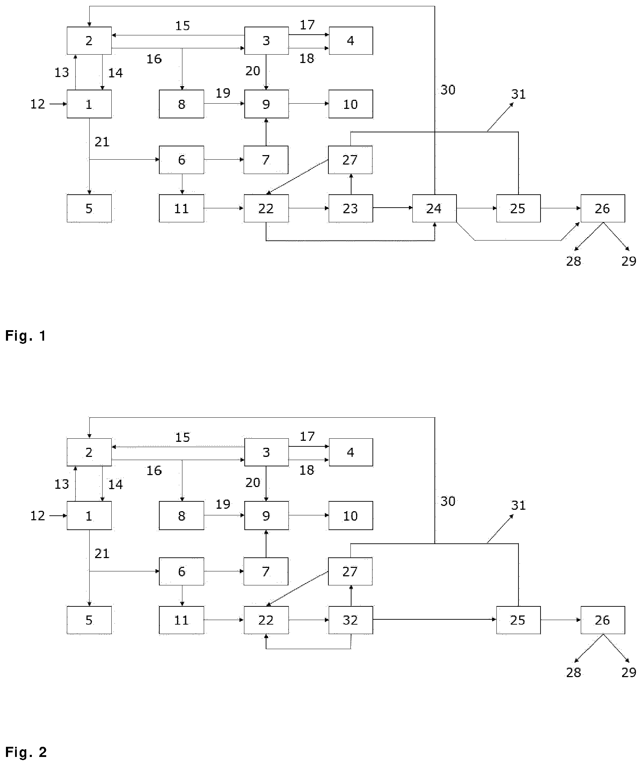

**Figure 1** shows a schematic representation of an arrangement according to an embodiment of the present invention.

Biomass (12), such as for instance manure and/or vegetable waste and/or fruit waste, is preheated by means of a heat exchanger (1) by hot digestate (14) from a fermentation tank (2). The preheated biomass (13) is fermented in the fermentation tank (2) by a thermophilic process, wherein the hot digestate (14) and biogas (16) are formed. The biogas (16) is used as fuel for a gas engine of a cogeneration installation (3). The cogeneration installation (3) produces heat (17) and electricity (18) as an energy source for activities (4) in, for example, a company, farm or house. Any surplus of heat (17) and electricity (18) can be sold. The electricity (18) is also used as an energy source for the arrangement itself. The gas engine of the cogeneration installation (3) comprises a cooling circuit for cooling the gas engine in operation. The fermentation tank (2) comprises heating pipes. The heating pipes of the fermentation tank (2) are coupled to the cooling circuit of the gas engine of the cogeneration installation (3). As a result, the fermentation tank (2) can be heated by heat (15) from the cogeneration installation (3) and kept at the right temperature. The heat (15) would otherwise be absorbed and dissipated by the cooling circuit of the gas engine.

Cooled digestate (21) from the heat exchanger (1) is stored as valuable raw material in a storage tank (5). Preferably, the cooled digestate (21) is separated into a first thick fraction (7) and a first thin fraction (11) using a separator (6). The first thick fraction (7) is dried into dried thick fraction (9). This is done, for example, by means of a drying auger. The drying auger uses heat (20), produced by the cogeneration installation (3), for example hot flue gases from the gas engine. Alternatively, if for example there is a surplus of biogas (16), biogas (16) is burned in a burner (8) and heat (19) from the burner (8) is used for drying the first thick fraction (7) into a dried thick fraction (9), for example by means of a hot air blower or by means of the said drying auger. The dried thick fraction (9) is pressed into pellets using a pellet press (10). The pellets are a valuable product, namely fertilizer, and can be spread locally on fields or sold as fertilizer.

The thin fraction (11) is placed in an aeration tank (22). In the aeration tank (22), nitrogen is removed from the thin fraction (11). From the aeration tank (22), a thin fraction is placed in an optional settling tank (23), in which sludge settles. The sludge is separated in a separator (27), into a second thin fraction and a second thick fraction. The second thin fraction flows back to the aeration tank (22). The thin fraction is further purified after the settling tank (23) in a dissolved air flotation installation (24) and an optional ultrafiltration installation (25). If the optional settling tank (23) is not present, the thin fraction from the aeration tank (22) is fed directly to the dissolved air flotation installation (24). Sludge from the dissolved air flotation installation (24) and the ultrafiltration installation (25), together with the second thick fraction, are returned as biomass (30) to the fermentation tank (2). Alternatively, sludge from the settling tank (23) is not separated into a second thin fraction and a second thick fraction using a separator (27) but directly returned, together with sludge from the dissolved air flotation installation (24) and the ultrafiltration installation (25) as biomass (30) to the fermentation tank (2). This possibility is not shown in Figure 1. Alternatively, sludge (31) is carried away for further processing.

The thin fraction is finally separated into water (28) in a reverse osmosis installation (26) that can be discharged or used as irrigation water or process water, and into mineral concentrate (29). If the optional ultrafiltration installation (25) is not present, liquid from the dissolved air flotation installation (24) is fed directly to the reverse osmosis installation (26).

It will be apparent to one skilled in the art that thin fraction in this specification refers to both the first thin fraction (11) and the second thin fraction.

An arrangement as shown in Figure 1 is advantageous with a mineral concentrate (29), which is desirably mainly rich in potassium.

**Figure 2** shows a schematic representation of an arrangement according to an alternative embodiment of the present invention.

This arrangement is substantially similar to an arrangement as shown in Figure 1.

The difference is that thin fraction from the aeration tank (22) is introduced into a membrane bioreactor (32). Sludge from the membrane bioreactor (32) is separated into a second thin fraction and a second thick fraction by means of a separator (27). The second thin fraction flows back to the aeration tank (22). At high flow rates it may not be possible to separate all sludge using the separator (27). In that case, part of the sludge is returned to the aeration tank (22) via an overflow from the membrane bioreactor (32). The thin fraction is further purified after the membrane bioreactor (32) in an ultrafiltration installation (25). Sludge from the ultrafiltration installation (25) is returned to the fermentation tank (2) as biomass (30) together with the second thick fraction. Alternatively, sludge (31) is carried away for further processing. The thin fraction is finally separated into water (28) in a reverse osmosis installation (26) that can be discharged or used as irrigation water or process water, and into mineral concentrate (29).

It will be apparent to one skilled in the art that thin fraction in this specification refers to both the first thin fraction (11) and the second thin fraction.

An arrangement as shown in Figure 2 is advantageous with a mineral concentrate (29), which is desirably mainly rich in potassium.

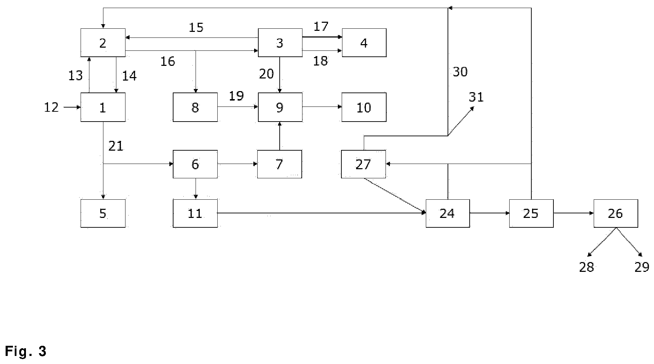

**Figure 3** shows a schematic representation of an arrangement according to yet another alternative embodiment of the present invention.

This arrangement is substantially similar to an arrangement as shown in Figure 1.

The difference is that the first thin fraction (11) is purified directly in a dissolved air flotation installation (24) and an ultrafiltration installation (25). Sludge from the dissolved air flotation installation (24) and the ultrafiltration installation (25) is separated into a second thin fraction and a second thick fraction using a separator (27). The second thin fraction is returned to the dissolved air flotation installation (24). The second thick fraction is returned to the fermentation tank (2) as biomass (30). Alternatively, sludge (31) is carried away for further processing. Alternatively, sludge from the dissolved air flotation installation (24) and the ultrafiltration installation (25) is returned directly to the fermentation tank (2). The thin fraction is finally separated into water (28) in a reverse osmosis installation (26) that can be discharged or used as irrigation water or process water, and into mineral concentrate (29).

It will be apparent to one skilled in the art that thin fraction in this specification refers to both the first thin fraction (11) and the second thin fraction.

An arrangement as shown in Figure 3 is advantageous with a mineral concentrate (29), which is desirably mainly rich in nitrogen and potassium.

## Claims

1. Arrangement for producing electricity and heat comprising a fermentation tank, wherein the fermentation tank comprises an insulated bottom, insulated side walls, a gastight roof, a biomass inlet, a biogas outlet, a digestate outlet and heating pipes, and a cogeneration installation, comprising a gas engine and a generator, wherein the gas engine comprises a gas inlet and a motor shaft, the generator comprising a generator shaft, wherein the gas inlet is coupled to the biogas outlet and wherein the motor shaft is coupled to the generator shaft, **characterized in that** the gas engine comprises a cooling circuit, wherein the heating pipes of the fermentation tank are coupled to the cooling circuit of the gas engine.

2. Arrangement according to claim 1, **characterized in that** the insulated bottom and the insulated side walls of the fermentation tank define an interior space, wherein the bottom and the side walls are insulated on a side facing the interior space.

3. Arrangement according to claim 1 or 2, **characterized in that** the arrangement comprises a heat exchanger configured for exchanging heat between two separate circuits, wherein a first circuit is connected to the biomass inlet of the fermentation tank and a second circuit is connected to the digestate outlet.

4. Arrangement according to any of the preceding claims 1-3, **characterized in that** the gas engine comprises a valve control and that the arrangement comprises a gas sensor, wherein the gas sensor is configured to measure an amount of methane in the biogas and wherein the valve sensor is communicatively connected to the gas sensor.

5. Arrangement according to any of the preceding claims 1-4, **characterized in that** the arrangement comprises a separator configured to separate the digestate into a first thin fraction and a first thick fraction, wherein the first thin fraction has a dry matter content of at most 50 kg/m³ and wherein the first thick fraction has a dry matter content of at least 200 kg/m³.

6. Arrangement according to claim 5, **characterized in that** the arrangement comprises a purification installation configured to purify the first thin fraction, the purification installation comprising a dissolved air flotation installation, an optional ultrafiltration installation and a reverse osmosis installation.

7. Arrangement according to claim 6, **characterized in that** the purification installation comprises a separator configured for separating sludge from the purification installation into a second thin and a second thick fraction, the separator comprising a return pipe for the second thin fraction to the purification installation, wherein the second thin fraction has a dry matter content of at most 50 kg/m³ and wherein the second thick fraction has a dry matter content of at least 200 kg/m³.

8. Arrangement according to any of the preceding claims 1-7, **characterized in that** the arrangement comprises a separator configured for separating the biomass into a third thin fraction and a third thick fraction, wherein the third thin fraction has a dry matter content of at most 50 kg/m³ and wherein the third thick fraction has a dry matter content of at least 200 kg/m³.

9. Method for producing electricity and heat comprising
- feeding biomass into a fermentation tank;
- fermentation of the biomass in the fermentation tank for at least fifty days at a temperature of at least 48°C and at most 55°C;
- discharging digestate from the fermentation tank;
- discharging biogas from the fermentation tank;
- generating heat and electricity from the biogas in a cogeneration installation, comprising a gas engine and a generator;
**characterized in that** the fermentation tank is heated with cooling water from the gas engine.

10. Method according to claim 9, **characterized in that** the method comprises the additional step of preheating the biomass by the digestate by means of a heat exchanger before feeding it into the fermentation tank.

11. Method according to claim 9 or 10, **characterized in that** the method comprises the additional step of separating the digestate using a separator into a first thin fraction and a first thick fraction, wherein the first thin fraction has a dry matter content of at most 50 kg/m³ and wherein the first thick fraction has a dry matter content of at least 200 kg/m³.

12. Method according to claim 11, **characterized in that** the method comprises the additional step of purifying the first thin fraction in a purification installation, wherein water, mineral concentrate and a residual fraction are obtained.

13. Method according to claim 12, **characterized in that** the method comprises the additional step of returning the residual fraction to the fermentation tank.

14. Method according to any of the preceding claims 9-13, **characterized in that** the method comprises the additional step of separating the biomass using a separator into a third thin fraction and a third thick fraction if the biomass has a dry matter content of less than 150 kg/m³, wherein the third thin fraction has a dry matter content of at most 50 kg/m³ and wherein the third thick fraction has a dry matter content of at least 200 kg/m³.

15. Use of the arrangement according to any of claims 1-8 or the method according to any of claims 9-14 for production in an industrial company of electricity and heat from biomass.
